(19) Europäisches Patentamt
European Patent Office
Office européen des brevets

(11) Veröffentlichungsnummer: **0 224 902**
**B1**

# (12) EUROPÄISCHE PATENTSCHRIFT

(45) Veröffentlichungstag der Patentschrift: **12.04.89**

(51) Int. Cl.⁴: **C 07 C 147/06**

(21) Anmeldenummer: **86116667.6**

(22) Anmeldetag: **01.12.86**

(54) **Verbesserte Verfahren zur Herstellung von D-threo-1-(p-Methylsulfonylphenyl)-2-dichloracetamido-propandiol-1,3-(Thiamphenicol)sowie Verwendung geeigneter Zwischenprodukte.**

(30) Priorität: **05.12.85 DE 3543021**

(43) Veröffentlichungstag der Anmeldung: **10.06.87 Patentblatt 87/24**

(45) Bekanntmachung des Hinweises auf die Patenterteilung: **12.04.89 Patentblatt 89/15**

(84) Benannte Vertragsstaaten: **AT BE CH DE ES FR GB GR IT LI LU NL SE**

(56) Entgegenhaltungen:
**EP-A- 0 014 437**
**DE-A- 2 454 805**
**US-A- 2 742 500**

**JOURNAL OF THE AMERICAN CHEMICAL SOCIETY, Band 77, Nr. 1, 12. Januar 1955, Seiten 186-187, US; M.C. REBSTOCK et al.: "The resolution of DL-threo-1-p-Methylsulfonyl-phenyl-2-amino-1,3-propanediol"**

(73) Patentinhaber: **BOEHRINGER MANNHEIM GMBH, Sandhofer Strasse 116, D-6800 Mannheim 31 (DE)**

(72) Erfinder: **Dick, Helmut, Dr.rer.nat., Kalmitstrasse 21, D-6710 Frankenthal (DE)**
Erfinder: **Gradel, Wolf-Dietrich, Dr.rer.nat., Richinesstrasse 4, D-6700 Ludwigshafen-Ruchheim (DE)**
Erfinder: **Weber, Mathias, Dr.rer.nat., Schönfelderstrasse 4, D-6704 Mutterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft verbesserte Verfahren zur Herstellung von Thiamphenicol, insbesondere betrifft sie die Racematenspaltung von Thiamphenicol-Vorstufen sowie Zwischenprodukte, die in diesen Verfahren einsetzbar sind.

Thiamphenicol ist eine optisch aktive Verbindung, die eine etwa doppelt so starke antibakterielle Aktivität besitzt wie die entsprechende racemische Verbindung (M.C. Rebstock, L.L. Bambas, Am. Soc. *77*, 186 [1955]).

Wichtiges Zwischenprodukt bei der Synthese von Thiamphenicol (I) ist das D,L-threo-1-(p-Methylsulfonylphenyl)-2-amino-propandiol-1,3(D,L-threo-Aminodiol) (II). Dieses wird durch Hydrierung des in Analogie zum Verfahren der DE-B-862 302 durch Kondensation von p-Methylsulfonylbenzaldehyd mit 2-Nitroethanol herstellbaren D,L-threo-1(p-methylsulfonylphenyl)-2-nitropropandiol-1,3(SNK) mit Pd/C als Katalysator in Form des Sulfats (III) erhalten.

Für die Enantiomeren-Spaltung sind aus der Literatur verschiedene Verfahren auch bei ähnlichen Verbindungen bekannt. Üblicherweise wird dabei das Enantiomeren-Gemisch mit optisch aktiven Hilfsreagenzien umgesetzt und die so erzeugten diastereomeren Addukte aufgrund ihrer unterschiedlichen physikalischen Eigenschaften aufgespalten (vgl. z. B. US-B-2,742,500). In selteneren Fällen gelingt es auch, die Enantiomeren direkt durch selektive Kristallisation aus dem Racemat zu gewinnen. So ist dies inzwischen bei einer ganzen Anzahl von Verbindungen gelungen (vgl. Chem. Reviews *63*, 297 [1963]). Eine Übertragung auf die Herstellung von Thiamphenicol (I) aus dem D,L-threo-Aminodiol (II) ist jedoch bisher nicht bekanntgeworden. Dies hat offenbar die inzwischen experimentell belegte Ursache, dass die freie Base (II) im Unterschied zum Beispiel zu der entsprechenden freien Base bei der Chloramphenicolsynthese nicht selektiv zur Kristallisation zu bringen ist.

Aufgabe der vorliegenden Erfindung war es daher, Verfahren zu entwickeln, mit denen die Racematenspaltung beim Thiamphenicolverfahren durch selektive Kristallisation, die der Trennung mit Hilfsreagenzien ökonomisch und ökologisch überlegen ist, durchgeführt werden kann. Dabei war es insbesondere ein Ziel, diese selektive Kristallisation in ihrer wirtschaftlichsten Form, der sogenannten Schaukelracematentrennung durchzuführen. Unter Schaukelracematentrennung versteht man eine Racematenspaltung durch wechselseitige selektive Kristallisation der Enantiomeren aus einer übersättigten Lösung des Racemates nach Animpfen (vgl. R.M. Secor: Resolution of Optical Isomers by Crystallisation Procedures in Chem. Reviews *63*, 297 [1963]).

Diese Aufgabe wird gemäss der Erfindung dadurch gelöst, dass

a) eine für die selektive Kristallisation geeignete Zwischenstufe ausgewählt,

b) besonders geeignete Lösungsmittel für die selektive Kristallisation gefunden, und

c) ein wirtschaftliches Verfahren zur Herstellung von Thiamphenicol aus der Zwischenstufe entwickelt wurden.

Gegenüber den aus der Literatur bekannten Verfahren mit optisch aktiven Hilfsstoffen führt das neue Verfahren zu einer dratischen Verringerung der Produktionskosten, da die teuren Hilfsstoffe entfallen und die Ausbeute nahezu quantitativ ist. Darüber hinaus ermöglicht das Verfahren die Isolierung auch des „falschen" Isomeren (L-threo-Verbindung) und eröffnet dadurch die Möglichkeit der Wiederverwendung, z. B. erneute Racemisierung und Rückführung in das Trennverfahren.

Ausgehend von D,L-threo-1(p-Methylsulfonylphenyl)-2-amino-propandiol-1,3-hydrochlorid (V), das aus dem Sulfat (III) in an sich bekannter Weise über die Schiff'sche Base (IV) und deren Hydrolyse mit Salzsäure gewonnen werden kann, verlaufen die Racematenspaltung sowie die Umsetzung zum fertigen Produkt nach folgendem Schema

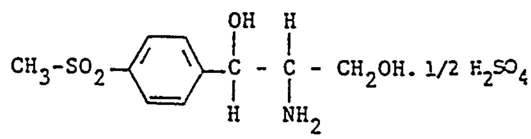
(III)

D,L-threo-1(p-Methylsulfonylphenyl)-2-amino-propandiol-1,3-sulfat („D,L-threo-Aminodiolsulfat", „Hydrierlösung")

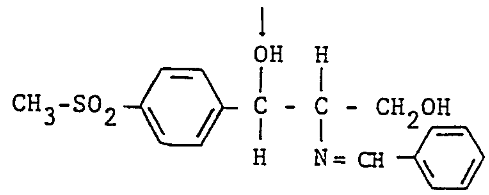
(IV)

„D,L-threo-Aminodiol-benzalverbindung"

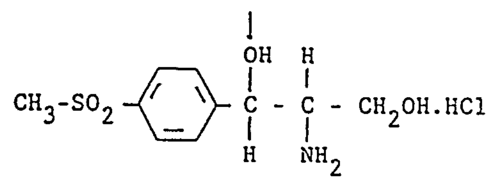
(V)

„D,L-threo-Aminodiol · HCl"

(a) (Racematentrennung)

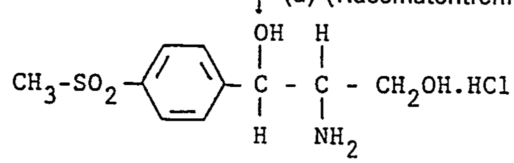
(VI)

„D-threo-Aminodiol · HCl" + L-Verbindung (VII)

(b)

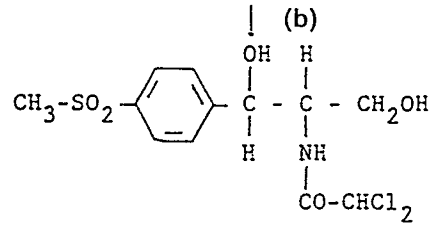
(I)

Thiamphenicol.

Es hat sich überraschenderweise herausgestellt, dass das Hydrochlorid (V) im Unterschied zu der freien Base (II) und zu einer Vielzahl von Salzen mit anorganischen und organischen Säuren hervorragend eine selektive Kristallisation auf der Stufe (a) zulässt.

Die selektive Kristallisation bzw. vorzugsweise die Schaukelracematenspaltung auf Stufe (a) wird vorzugsweise aus übersättigten Lösungen in Gemischen aus Alkanol/Wasser, Alkanol/Wasser/konz. Salzsäure, Wasser/konz. Salzsäure oder Alkanol/Chlorwasserstoff in beliebigen Molverhältnissen durchgeführt. Unter Alkanolen sind vorzugsweise niedere Alkohole zu verstehen. Insbesondere Ethanol und Methanol. Die Konzentration der entsprechenden Aminodiol-HCl-Verbindungen (V, VI, VII) in der Kristallisationslösung bewegt sich bei Kristallisationstemperatur bis 200% Übersättigung, besonders bevorzugt bis 150% Übersättigung. Die selektive Kristallisation ist zwischen −20 und +80° C durchführbar. Vorzugsweise liegt die Kristallisationstemperatur zwischen 0 und 60° C.

Die Weiterverarbeitung (Stufe b) des durch die Enantiomerentrennung gewonnenen D-threo-Aminodiol · HCl (VI) (vgl. GB-B-745.900) zum Thiamphenicol (I) geschieht mit einer Ausbeute von ca. 96% (einschl. Rückläufen), indem das D-threo-Aminodiol · HCl (VI) in Methanol mit Natriummethylat in die freie Base überführt, mit Dichloressigsäuremethylester zu Thiamphenicol umgesetzt und das Thiamphenicol aus Methanol/Wasser kristallisiert wird.

Die optische Reinheit des bei der Schaukelracematentrennung anfallenden D-threo-Aminodiol · HCl (VI) genügt im allgemeinen für die Thiamphenicolherstellung. In Ausnahmefällen kann der Gehalt an falschem Isomeren zu hoch sein. Hier kann durch nachträgliches Ausrühren des D-threo-Aminodiol · HCl aus 95%igem Alkohol oder aus verdünnter wässeriger Salzsäure die gewünschte optische Reinheit leicht erreicht werden.

Das Verfahren zur Herstellung von Thiamphenicol aus D-threo-Aminodiol · HCl mit Natriummethylat und Dichloressigsäuremethylester in Methanol wird besonders vorteilhaft so durchgeführt, dass die Umsetzung in Edelstahlgefässen unter Zusatz von Ethylen-diamin-tetra-essigsäure-tetranatriumsalz erfolgt.

In den nachfolgenden Beispielen wird das Verfahren näher erläutert:

*Beispiel 1*

900 g D,L-threo-1(p-Methylsulfonylphenyl)-2-aminopropandiol-1,3-hydrochlorid (D,L-threo-Aminodiol · HCl) (V) (Fp 172-175° C) und 90 g L-threo-Aminodiol · HCl ($[\alpha]_D^{20}$ + 26,5 (c = 2, $H_2O$); Fp 201-203° C) werden in 4050 g Ethanol (95%ig, vergällt mit 1% Methylethylketon) unter Rückfluss gelöst, mit 20 g Aktivkohle 15 min unter Rückfluss erhitzt und filtriert. Das Filtrat wird mit Ethanol auf ein Gewicht von 5040 g eingestellt (= 6020 ml bei 50° C). Man erhält eine bei 50° C übersättigte alkoholische Lösung von folgendem Gehalt:

17,9% D,L-threo-Aminodiol · HCl, und
1,8% L-threo-Aminodiol · HCl

Unter langsamem Rühren wird bei 50° C und einer Anfangsdrehung der Lösung $\alpha_D^{50}$ von +0,8° mit 0,5 g L-threo-Aminodiol · HCl ($[\alpha]_D^{20}$ + 26,5 (c = 2, $H_2O$), Fp 201-203° C; Kristallgrösse 98% < 74 μ) angeimpft. Um den Verlauf und den Abschluss der Kristallisation beobachten zu können, werden von Zeit zu Zeit filtrierte Proben im Polarimeter ($\alpha_D^{50}$ in 2-dm-Küvette) gemessen. Nach einer Kristallisationszeit von ca. 4 Stdn. wird bei einer Enddrehung der filtrierten Lösung von −0,9° abgesaugt, das Kristallisat mit 300 ml Ethanol (95%ig) von 0° C gewaschen und bei 50° C getrocknet. Ausbeute: 170 g (die Ausbeute an aktivem Salz liegt wegen der Probenahmen [ca. 5 mit Messung $\alpha_D^{50}$] für die Inprozesskontrolle unter der erwarteten Ausbeute von ca. 180 g) L-threo-Aminodiol · HCl (VII) ($[\alpha]_D^{20}$ = +25,0 (c = 2, $H_2O$); Fp 200-202° C).

Man vereinigt Waschflüssigkeit und Mutterlauge, fügt 180 g D,L-threo-Aminodiol · HCl hinzu, löst, kohlt und stellt die Konzentration von ca. 17,9% D,L- und 1,8% D-Verbindung durch destillation ein. Nach dem Animpfen mit 0,5 g D-threo-Aminodiol · HCl bei 50° C wird wieder ca. 4 Stdn. kristallisiert, bis die optische Drehung der filtrierten Lösung $\alpha_D^{50}$ auf +0,9° gestiegen ist.

Man saugt ab, wäscht mit 300 ml Ethanol (95%ig) und trocknet bei 50° C. Ausbeute:. 170 g D-threo-Aminodiol · HCl (VI) ($[\alpha]_D^{20}$ = −25,0 (c = 2, $H_2O$); Fp 200-202° C).

Als nächstes folgt wieder eine Kristallisation von L-threo-Aminodiol · HCl (VII) usw.

Man kann die Kristallisation sehr oft wiederholen. Wegen der sich in der Mutterlauge anreichernden Verunreinigungen erhöhen sich die Löslichkeiten und damit die Kristallisationszeiten. Damit diese konstant bleiben, kann man 1. die Kristallisationstemperatur allmählich von Ansatz zu Ansatz bis auf ca. +30° C senken und/oder 2. die Konzentration an D,L-threo-Aminodiol · HCl allmählich von Ansatz zu Ansatz erhöhen.

*Beispiel 2*

1800 g D,L-threo-1(p-methylsulfonylphenyl)-2-aminopropandiol-1,3-hydrochlorid (D,L-threo-Aminodiol · HCl) (V) (Fp 172-175° C) und 150 g L-threo-Aminodiol · HCl ($[\alpha]_D^{20}$ +26,5° (c = 2, $H_2O$); Fp 201-203° C) werden in 1150 ml destilliertem Wasser bei 50° C gelöst und die Lösung filtriert. Das Filtrat wird mit 1130 g 38%iger Salzsäure versetzt und auf 30° C abgekühlt.

Man erhält eine übersättigte wässerige Lösung mit folgendem Gehalt:

42,6% D,L-threo-Aminodiol · HCl
3,5% L-threo-Aminodiol · HCl
10,2% Chlorwasserstoff

Die optische Drehung $\alpha_D^{30}$ einer Probe – 1:5 mit Wasser verdünnt und in einer 1-dm-Küvette mit dem Polarimeter gemessen – beträgt +0,5°. Bei 30° C impft man mit 1 g L-threo-Aminodiol · HCl (Kristallgrösse 98% < 74 μ) an und kristallisiert ca. 6 Stunden unter mässigem Rühren bei 30° C, bis die

optische Drehung $\alpha_D^{30}$ einer filtrierten Probe (1:5 mit Wasser verdünnt) −0,6° beträgt.

Man saugt ab und wäscht portionsweise mit 260 ml Ethanol (95%ig) von 30° C und dann mit 780 ml Ethanol (95%ig) von 0-5° C. Das Kristallisat (L-threo-Aminodiol·HCl) (VII) wiegt nach dem Trocknen bei 60° C 290 g (die Ausbeute an aktivem Salt liegt wegen der Probenahmen für die Inprozesskontrolle unter der erwarteten Ausbeute von ca. 300 g) ($[\alpha]_D^{20}$ +25,5° (c = 2, $H_2O$); Fp 201-202° C). Nach dem Einengen des Waschethanols erhält man 150 g nahezu racemisches Aminodiol·HCl. Man löst den Rückstand aus der Waschflüssigkeit in der Mutterlauge der L-Kristallisation, stockt mit 300 g D,L-threo-Aminodiol·HCl auf, löst bei ca. 70° C und stellt die Konzentrationsverhältnisse wie vor Beginn der L-Kristallisation her.

Nach dem Animpfen mit 1 g D-threo-Aminodiol·HCl (VI) bei 30° C wird wieder ca. 6 Stdn. langsam gerührt, bis die optische Drehung $\alpha_D^{30}$ von −0,5° auf +0,6° gestiegen ist.

Man saugt ab, wäscht mit Ethanol (95%ig) und trocknet das D-threo-Aminodiol·HCl (VI) bei 60° C. Ausbeute: 290 g (die Ausbeute an aktivem Salz wegen der Probenahmen für die Inprozesskontrolle unter der erwarteten Ausbeute von ca. 300 g) ($[\alpha]_D^{20}$ −25,5° (c = 2, $H_2O$); Fp 201-202° C).

Als nächstes folgt wieder eine Kristallisation von L-threo-Aminodiol·HCl (VII) usw.

Je nach Reinheit des eingesetzten D,L-threo-Aminodiol·HCl (V) kann aus derselben Lösung bis zu 100mal und mehr abwechselnd L- und D-Verbindung kristallisiert werden. Die Kristallisationszeiten nehmen dabei von Ansatz zu Ansatz langsam zu. Durch allmähliche Erhöhung der Konzentration von D,L-threo-Aminodiol·HCl (bis zu ca. 52%) und der Konzentration von Chlorwasserstoff auf ca. 12% können die Kristallisationszeiten zwischen 3 und 10 Stdn. gehalten werden.

*Beispiel 3*

1168 g D,L-threo-1(p-methylsulfonylphenyl)-2-amino-propandiol-1,3-hydrochlorid (D,L-threo-Aminodiol·HCl) (V) (Fp 172-175° C) und 85 g L-threo-Aminodiol·HCl ($[\alpha]_D^{20}$ +26,5° (c = 2, $H_2O$); Fp 201-203° C) werden in einem Gemisch aus 379 g Wasser und 734 g Ethanol (99%ig, vergällt mit 1% Methylethylketon) unter Rückfluss gelöst. Nach Abkühlung auf 25° C erhält man eine übersättigte Lösung mit folgendem Gehalt:

49,4% D,L-threo-Aminodiol·HCl
3,6% L-threo-Aminodiol·HCl
16,0% Wasser
31,0% Ethanol (99%ig, vergällt mit 1% Methylethylketon)

Die optische Drehung $\alpha_D^{25}$ einer Probe – in einer 1-dm-Küvette mit dem Polarimeter gemessen – beträgt +1,2°. Bei 25° C impft man mit 1 g L-threo-Aminodiol·HCl (Kristallgrösse 98% <74 µ) an und kristallisiert ca. 6 Stunden unter mässigem Rühren bei 25° C, bis die optische Drehung $\alpha_D^{25}$ einer filtrierten Probe −1,3° beträgt.

Man saugt ab und wäscht portionsweise einmal mit 260 ml Ethanol (95%, vergällt) von 40° C und dreimal mit je 260 ml Ethanol (95%, vergällt) von 0-5° C. Das Kristallisat (L-threo-Aminodiol·HCl) (VII) wiegt nach dem Trocknen bei 60° C 160 g (die Ausbeute an aktivem Salz liegt wegen der Probenahmen für die Inprozesskontrolle unter der erwarteten Ausbeute von ca. 170 g) ($[\alpha]_D^{20}$ +25,3° (c = 2, $H_2O$); Fp 200,5-201,5° C).

Nach dem Einengen des Waschethanols erhält man 190 g nahezu racemisches Aminodiol·HCl. Man löst den Rückstand aus der Waschflüssigkeit in der Mutterlauge der L-Kristallisation, stockt mit 170 g D,L-threo-Aminodiol·HCl auf, löst bei Rückfluss und stellt die Konzentrationsverhältnisse durch Zugabe vor Ethanol und Wasser wie vor Beginn der L-Kristallisation her.

Nach dem Animpfen mit 1 g D-threo-Aminodiol·HCl (VI), bei 25° C, wird wieder ca. 6 Stunden langsam gerührt, bis die optische Drehung $\alpha_D^{25}$ von −1,2° auf +1,3° gestiegen ist.

Man saugt ab, wäscht wie oben geschildert mit Ethanol (95%ig) und trocknet das D-threo-Aminodiol·HCl (VI) bei 60° C.

Ausbeute: 160 g (die Ausbeute an aktivem Salz liegt wegen der Probenahmen für die Inprozesskontrolle unter der erwarteten Ausbeute von ca. 170 g) ($[\alpha]_D^{20}$ −25,5° (c = 2, $H_2O$); Fp 201-202° C).

Als nächstes folgt wieder eine Kristallisation von L-threo-Aminodiol·HCl (VII) usw.

## Patentansprüche

1. Verfahren zur Herstellung von Thiamphenicol (I) aus D,L-threo-1(p-Methylsulfonylphenyl)-2-amino-propandiol-1,3-hydrochlorid (V), dadurch gekennzeichnet, dass es folgende Stufen umfasst:

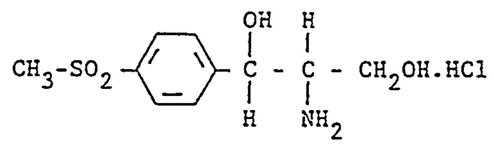

(V)

„D,L-threo-Aminodiol·HCl"

(a) (Racematentrennung)

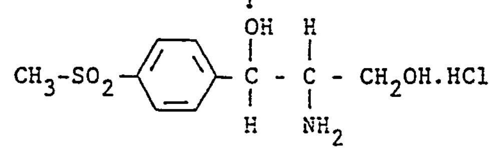

(VI)

D-threo-Aminodiol·HCl

(b)

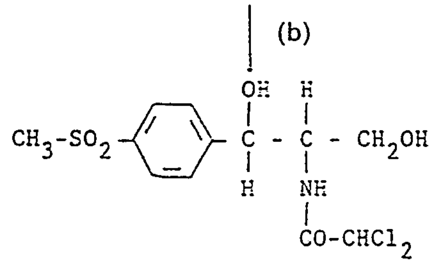

(I)

Thiamphenicol.

2. Verfahren zur Herstellung von

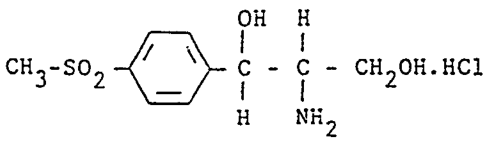

(VI)

D-threo-Aminodiol · HCl
aus racemischem D,L-threo-Aminodiol · HCl (V) durch selektive Kristallisation aus einer übersättigten Lösung durch Animpfen mit D-threo-Aminodiol · HCl, dadurch gekennzeichnet, dass die Lösung bis zu 200% an D,L-threo-Aminodiol · HCl sowie an D-threo-Aminodiol · HCl übersättigt ist, das Lösungsmittel ein niederer Alkohol, ggf. unter Zusatz von Chlorwasserstoff, ein Gemisch aus einem niederen Alkohol und Wasser, ggf. unter Zusatz von konz. Salzsäure, oder wässerige Salzsäure ist und die Kristallisationstemperatur zwischen −20 und +80° C liegt.

3. Verfahren zur Herstellung von

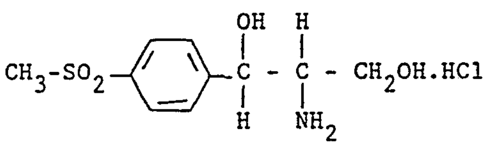

(VII)

L-threo-Aminodiol · HCl
aus racemischem D,L-threo-Aminodiol · HCl (V) durch selektive Kristallisation aus einer übersättigten Lösung durch Animpfen mit L-threo-Aminodiol · HCl, dadurch gekennzeichnet, dass die Lösung bis zu 200% an D,L-threo-Aminodiol · HCl sowie an L-threo-Aminodiol · HCl übersättigt ist, das Lösungsmittel ein niederer Alkohol, ggf. unter Zusatz von Chlorwasserstoff, ein Gemisch aus einem niederen Alkohol und Wasser, ggf. unter Zusatz von konz. Salzsäure, oder wässerige Salzsäure ist und die Kristallisationstemperatur zwischen −20 und +80° C liegt.

4. Verfahren nach den Ansprüchen 2 und 3, dadurch gekennzeichnet, dass die selektive Kristallisation der beiden Enantiomeren (VI und VII) in Form einer Schaukelracematentrennung durchgeführt wird.

5. Verfahren zur Aufreinigung des mit L-Verbindung (VII) verunreinigten D-threo-Aminodiol · HCl (VI) gemäss Anspruch 2 durch Ausrühren oder Umkristallisation aus Alkoholen bzw. Alkohol-Wassergemischen mit und ohne Salzsäure oder wässerige Salzsäure.

6. Verfahren zur Herstellung von Thiamphenicol aus D-threo-Aminodiol · HCl mit Natriummethylat und Dichloressigsäuremethylester in Methanol, dadurch gekennzeichnet, dass die Umsetzung in Edelstahlgefässen unter Zusatz von Ethylendiamin-tetra-essigsäure-tetra-natriumsalz durchgeführt wird.

7. Verwendung von D,L-threo-Aminodiol · HCl (V) als Zwischenprodukt zur Herstellung von Thiamphenicol (I).

8. Verwendung von D-threo-Aminodiol · HCl (VI) als Zwischenprodukt zur Herstellung von Thiamphenicol (I).

9. D,L-threo-Aminodiol · HCl (V).

**Claims**

1. Process for the preparation of thiamphenicol (I) from D,L-threo-1-(*p*-methylsulphonylphenyl)-2-amino-propane-1,3-diol hydrochloride (V), characterised in that it comprises the following steps:

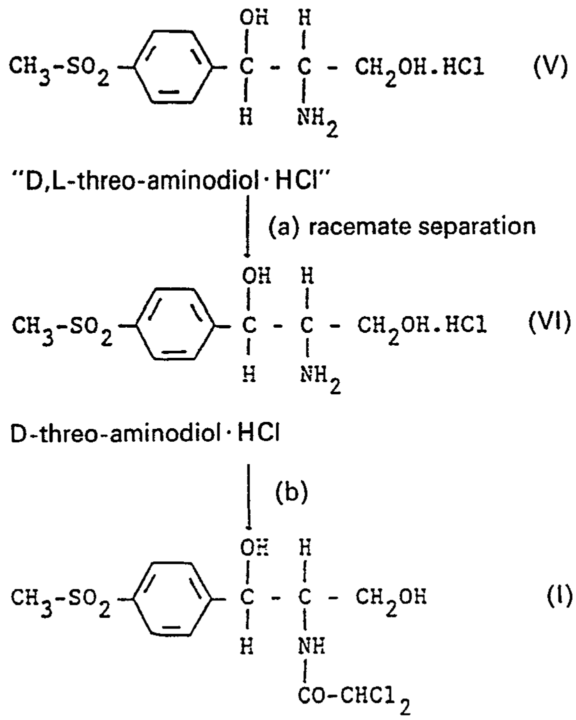


(V)

"D,L-threo-aminodiol · HCl"

(VI)

D-threo-aminodiol · HCl

(I)

thiamphenicol.

2. Process for the preparation of

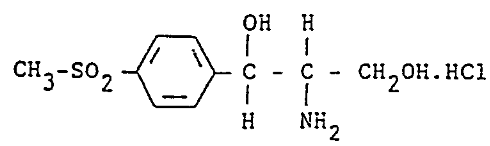

(VI)

D-threo-aminodiol · HCl
from racemic D,L-threo-aminodiol · HCl (V) by selective crystallisation from a supersaturated solution by seeding with D-threo-aminodiol · HCl, characterised in that the solution is supersaturated up to 200% of D,L-threo-aminodiol · HCl, as well as of D-threo-aminodiol · HCl, the solvent is a lower alcohol optionally with the addition of hydrogen chloride, a mixture of a lower alcohol and water optionally with the addition of conc. hydrochloric acid or aqueous hydrochloric acid and the crystallisation temperature lies between −20 and +80° C.

3. Process for the preparation of

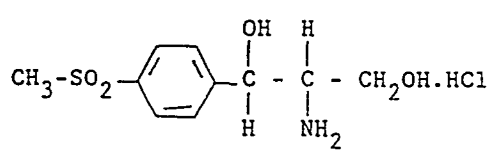

(VII)

L-threo-aminodiol · HCl
from racemic D,L-threo-aminodiol · HCl (V) by selective crystallisation from a supersaturated solution by seeding with L-threo-aminodiol · HCl, characterised in that the solution is supersaturated

up to 200% of D,L-threo-aminodiol · HCl, as well as of L-threo-aminodiol · HCl, the solvent is a lower alcohol optionally with the addition of hydrogen chloride, a mixture of a lower alcohol and water optionally with the addition of conc. hydrochloric acid or aqueous hydrochloric acid and the crystallisation temperature lies between −20 and +80° C.

4. Process according to claims 2 and 3, characterised in that the selective crystallisation of the two enantiomers (VI and VII) is carried out in the form of an oscillating racemate resolution.

5. Process for the purification of the D-threo-aminodiol · HCl (VI) contaminated with L-compound (VII) according to claim 2 by stirring up with or recrystallisation from alcohols or alcohol-water mixtures with and without hydrochloric acid or aqueous hydrochloric acid.

6. Process for the preparation of thiamphenicol from D-threo-aminodiol · HCl with sodium methylate and dichloroacetic acid methyl ester in methanol, characterised in that the reaction is carried out in stainless steel vessels with addition of ethylene-diamine-tetraacetic acid tetrasodium salt.

7. Use of D,L-threo-aminodiol · HCl (V) as intermediate product for the preparation of thiamphenicol (I).

8. Use of D-threo-aminodiol · HCl (VI) as intermediate product for the preparation of thiamphenicol (I).

9. D,L-threo-aminodiol · HCl (V).

## Revendications

1. Procédé de préparation de thiamphénicol (I) à partir du chlorhydrate de D,L-threo-1-(p-méthylsulfonylphényl)-2-amino-propanediol-1,3 (V), caractérisé en ce qu'il comprend les étapes suivantes:

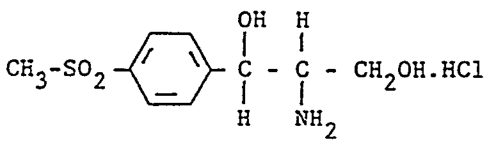
(V)

«D,L-threo-aminodiol · HCl»

(a)
(séparation de racémate)

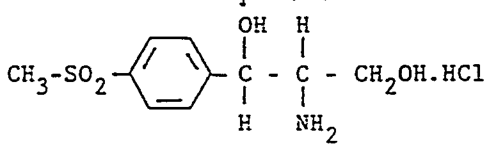
(VI)

D-threo-aminodiol · HCl

(b)

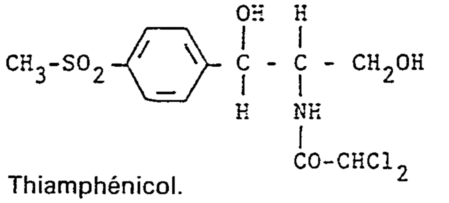
(I)

Thiamphénicol.

2. Procédé de préparation du

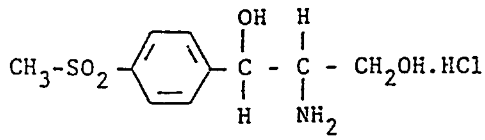
(VI)

D-threo-aminodiol · HCl

à partir de D,L-threo-aminodiol · HCl (V) racémique par cristallisation sélective à partir d'une solution saturée, en amorçant avec le D-threo-aminodiol · HCl, caractérisé en ce que la solution est sursaturée jusqu'à 200% en D,L-threo-aminodiol · HCl ainsi qu'en D-threo-aminodiol · HCl, que le solvant est un alcool inférieur, éventuellement avec addition de chlorure d'hydrogène, un mélange d'un alcool inférieur et d'eau, éventuellement avec addition d'acide chlorhydrique concentré, et que la température de cristallisation est comprise entre −20 et +80° C.

3. Procédé de préparation du

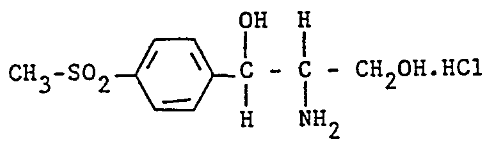
(VII)

L-threo-aminodiol · HCl

à partir de D,L-threo-aminodiol · HCl (V) racémique par cristallisation sélective à partir d'une solution saturée, en amorçant avec le L-threo-aminodiol · HCl, caractérisé en ce que la solution est sursaturée jusqu'à 200% en D,L-threo-aminodiol · HCl ainsi qu'en L-threo-aminodiol · HCl, que le solvant est un alcool inférieur, éventuellement avec addition de chlorure d'hydrogène, un mélange d'un alcool inférieur et d'eau, éventuellement avec addition d'acide chlorhydrique concentré, et que la température de cristallisation est comprise entre −20 et +80° C.

4. Procédé selon les revendications 2 et 3, caractérisé en ce que la cristallisation sélective des deux énantiomères (VI et VII) est effectuée sous la forme d'une séparation de racémate à oscillations.

5. Procédé pour la purification du D-threo-aminodiol · HCl (VI) contenant comme impureté le composé L (VII), selon la revendication 2, par agitation ou recristallisation dans un alcool ou un mélange eau-alcool, avec ou sans acide chlorhydrique ou acide chlorhydrique aqueux.

6. Procédé de préparation de thiamphénicol à partir de D-threo-aminodiol · HCl avec le méthylate de sodium et le méthylate de l'acide dichloracétique dans le méthanol, caractérisé en ce que la réaction est effectuée dans des récipients en acier inoxydable, avec addition du sel tétrasodique de l'acide éthylène-diaminetétracétique.

7. Utilisation du D,L-threo-aminodiol · HCl (V) comme produit intermédiaire dans la préparation du thiamphénicol (I).

8. Utilisation du D-threo-aminodiol · HCl (VI) comme produit intermédiaire dans la préparation du thiamphénicol (I).

9. D,L-threo-aminodiol · HCl (V).